(19) Europäisches Patentamt
European Patent Office
Office européen des brevets

(11) Numéro de publication: **0 099 822**
**B1**

(12) # FASCICULE DE BREVET EUROPÉEN

(45) Date de publication du fascicule du brevet: **05.11.86**

(21) Numéro de dépôt: **83401450.8**

(22) Date de dépôt: **13.07.83**

(51) Int. Cl.⁴: **C 07 D 333/38**, C 07 D 333/70, C 07 D 409/12, A 61 K 31/38 // (C07D409/12, 333:38, 303:12)

(54) **Nouveaux 1-(2-carbalkoxy 4-(thiénylalkylamido)phénoxy) 3-amino 2-propanols, leur préparation et leurs applications en thérapeutique.**

(30) Priorité: **16.07.82 FR 8212499**
**06.06.83 FR 8309361**

(43) Date de publication de la demande: **01.02.84 Bulletin 84/5**

(45) Mention de la délivrance du brevet: **05.11.86 Bulletin 86/45**

(84) Etats contractants désignés: **AT BE CH DE FR GB IT LI LU NL SE**

(56) Documents cité:
**EP-A-0 071 535**
**FR-A-1 543 689**
**FR-A-2 291 741**
**FR-A-2 458 548**

(73) Titulaire: **CARPIBEM (CENTRE D'ACTIVITE ET DE RECHERCHE PHARMACEUTIQUE INDUSTRIELLE BIOLOGIQUE ET MEDICALE) Société Anonyme dite:, 128, rue Danton, F-92500 Rueil- Malmaison (FR)**

(72) Inventeur: **Bouley, Etienne, 19, rue Barbès, F-92400 Courbevoie (FR)**
Inventeur: **Teulon, Jean- Marie, 12 Résidence du Bel Ebat, 56 Avenue de Verdun, F-78170 La Celle-Saint- Cloud (FR)**

(74) Mandataire: **Combe, André, CABINET BEAU DE LOMENIE 55 rue d'Amsterdam, F-75008 Paris (FR)**

**EP 0 099 822 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne en tant que produits nouveaux les composés de formule générale 1 ci-dessous et leurs sels d'addition d'acides. Les composés en question présentent un profil pharmacologique très original dans la mesure où ils sont doués de propriétés β-bloquantes comparables à celles du propranolol et de propriétés diurétiques comparables à celles des thiazidiques. La présente invention concerne également le procédé de préparation desdits produits et leurs applications en thérapeutique. Elle concerne en outre les composés intermédiaires nouveaux permettant la synthèse desdits produits.

On a déjà décrit dans le brevet français 2458548 des anilides d'acides (3-alkylamino-2-hydropropoxy)-furanne-2 carboxyliques ayant des propriétés β-bloquantes.

On a également décrit dans le brevet européen 71 535 des [2-ou 3-substitué 4-(thiényl-acétamido)phénoxy] hydroxy polyamines et on a indiqué que ces produits pouvaient être utiles comme ayant des propriétés β-bloquantes et donc antihypertensives.

On sait enfin qu'un certain nombre de médicaments à propriétés anti-hypertensives agissent en tant que diurétiques.

On a cherché, dans la présente invention, l'obtention d'un produit présentant à la fois des propriétés diurétiques et β-bloquantes et étant de ce fait particulièrement intéressant pour la lutte contre l'hypertension.

Les produits selon la présente invention répondent à la formule suivante:

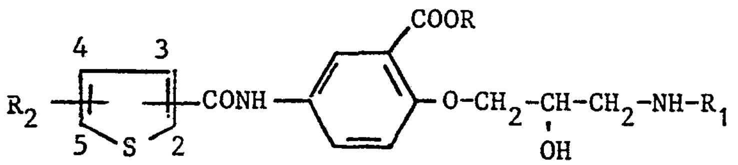

(I)

Dans la formule 1

- R représente un radical alkyle de 1 à 5 atomes de carbone, ramifié ou non, avantageusement le radical éthyle;
- $R_1$ est un radical alkyle de 1 à 5 atomes de carbone, linéaire ou ramifié et, avantageusement, $R_1$ est le radical isopropyle ou le radical terbutyle;
- $R_2$ peut être H, un radical alkyle de 1 à 5 atomes de carbone, linéaire, ramifié ou cyclique, ou un halogène et $R_2$ peut également désigner un cycle cyclohexyle ou le cylopentyle accolé au cycle thiophène, $R_2$ désignant avantageusement H ou le groupe méthyle;
- le groupe carboxamide étant lié au cycle thiophène en position 2 ou 3.

Les composés selon l'invention sont synthétisés par action d'une base $NH_2$-$R_1$ ($R_1$ étant défini comme ci-dessus) sur un composé de formule II, sans solvant ou dans un solvant organique usuel comme les alcools, à une température comprise entre 20° C et 150° C.

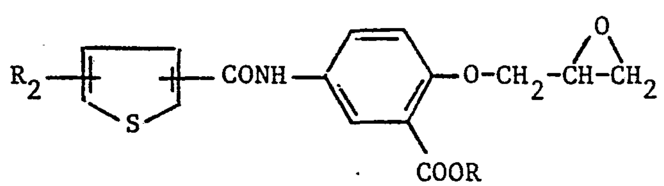

(II)

($R_2$ et R définis comme ci-dessus).

D'une façon générale, les composés de formule II peuvent être obtenus par réaction d'un phénol de formule générale III avec une épihalogénhydrine, notamment l'épichlorhydrine ou l'épibromhydrine. Le phénol de formule III est auparavant métallé par des agents usuels de métallation comme la soude, la potasse, un alcoolate ou l'hydrure de sodium, en milieu hydroalcoolique, alcoolique ou dans un solvant comme le DMF, à une température comprise entre 20° C et 150° C.

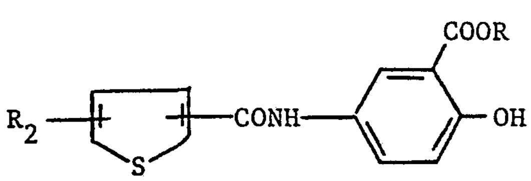

(III)

($R_2$ et R définis comme ci-dessus).

Ces procédés de synthèse connus en soi donnent des composés mal définis avec de faibles rendements. La demanderesse a découvert et mis au point un nouveau procédé consistant à faire réagir le phénol de formule III avec un excès d'épichlorhydrine en présence d'un catalyseur, comme le chlorure de benzyltriméthylammonium, à une température allant de 110°C à 130°C. Ce procédé fournit des composés cristallisés parfaitement définis avec de bons rendements.

Les composés de formule III sont obtenus par une méthode classique consistant à faire réagir le chlorure d'acide de l'acide thiényl carboxylique correspondant sur un 4-amino 2-carbalkoxy phénol, en présence d'une base comme la triéthylamine, dans un solvant comme l'acétone ou le benzène.

Les sels d'addition non toxiques des composés de formule I peuvent s'obtenir par réaction de ces composés avec un acide minéral ou organique suivant une méthode connue en soi. Parmi les acides utilisables à cet effet, nous citons les acides chlorhydrique, bromhydrique, sulfurique, phosphorique, 4-toluènesulfonique, méthanesulfonique, cyclohexylsulfamique, oxalique, succinique, formique, maléique, aspartique, cinnamique, glutamique, N-acétylaspartique, N-acétylglutamique, ascorbique, malique, fumarique, lactique et benzoïque.

Les nouveaux composés selon l'invention possèdent des propriétés pharmacologiques remarquables et peuvent être utilisés en thérapeutique comme β-bloqueurs de type propanolol et/ou comme diurétiques de type thiazidique, puisque, de manière surprenante, ils peuvent présenter simultanément ces deux propriétés, pour le traitement de l'hypertension. Ces produits sont d'autre part peu toxiques et cardiosélectifs.

Une sous-famille IA particulièrement préférée de composés selon l'invention répond à la formule (I) dans laquelle la chaine carboxamide est en position 2 de ce cycle. Les composés de cette famille présentent en effet simultanément d'excellentes propriétés diurétiques et β-bloquantes.

On notera encore que la sous-famille IB, caractérisée par le fait que la chaine carboxamide est en position 3, correspond à des produits associant une bonne activité β-bloquante à une tendance diurétique.

Par ailleurs, il a été constaté que les composés selon l'invention dans lesquels $R_2$ représente H ou méthyl-4 sont particulièrement intéressants. Ces produits sont notamment supérieurs à une substitution méthyl-5 sur le thiophène.

On notera donc l'intérêt tout particulier des composés dans lesquels la chaîne carboxamide est en position 2 du thiophène, et $R_2$ = H ou méthyl-4.

En thérapeutique humaine, les composés de formule 1 et leurs sels d'addition d'acides non toxiques peuvent être administrés notamment par voie orale. On préconise alors l'emploi de gélules ou de comprimés renfermant de 50 à 300 mg de principe actif en association avec un excipient physiologiquement acceptable. Les composés revendiqués présentent l'avantage de rendre le traitement plus aisé. D'autre part, par rapport aux associations β-bloqueurs/diurétiques utilisées dans le traitement de l'hypertension, les composés de formule IA ont l'avantage décisif d'une pharmacocinétique unique. Comme autres exemples d'indications possibles on citera l'angor, l'arythmie, les migraines.

D'autres caractéristiques et avantages de l'invention apparaîtront mieux à la lecture de quelques exemples de préparation non limitatifs donnés à titre d'illustration. Les formules correspondantes sont présentées dans le tableau unique ci-après.

**Exemple 1**

1-[2-carbéthoxy 4-(5-méthyl 2-thiophène carboxamido) phénoxy] 2,3-époxy propane.

Formule II R = $C_2H_5$ R = $CH_3$ en 5

Dans un ballon, le mélange de 35 g de 2-carbéthoxy 4-(5-méthyl 2-thiophéne carboxamido) phénol et 175 ml d'épichlorhydrine est chauffé à 110°C puis 2,9 g de chlorure de benzylthriméthylammonium sont introduits. Le mélange réactionnel est alors chauffé au reflux pendant 30 min puis refroidi. Une fois la température du milieu redescendue à 50°C on ajoute 200 ml d'eau et on agite fortement. Après décantation la phase aqueuse est extraite par deux fois 50 ml d'éther et les phases organiques sont séchées sur sulfate de magnésium filtrées et concentrées sous vide. Le résidu durcit dans l'éther. Après trois lavages par 50 ml d'éther isopropylique on obtient 30 g de 1-[2-carbéthoxy 4-(5-méthyl 2-thiophéne carboxamido) phénoxy] 2,3-époxy propane sous forme de cristaux blancs fondant à 109°C.

**Exemple 2**

1-[2-carbéthoxy 4-(5-méthyl 2-thiophéne carboxamido) phénoxy] 3-terbutylamino 2-propanol

$$\text{Formule I} \quad R = C_2H_5 \quad R_1 = -\overset{CH_3}{\underset{CH_3}{\overset{|}{\underset{|}{C}}}}-CH_3 \quad R_2 = CH_3 \text{ en } 5$$

Dans un ballon sont chauffés au reflux pendant 8 h 14 g de l'époxyde préparé à l'exemple 1, dans 30 ml de terbutylamine et 100 ml d'éthanol. La solution alcoolique est ensuite concentrée sous vide et le résidu repris dans 150 ml d'eau, 5 ml d'acide acétique glacial et 100 ml d'acétate d'isopropyle. La phase organique est éliminée par décantation et la phase aqueuse acide est neutralisée par une solution d'ammoniaque. Puis extraite par deux fois 50 ml de chloroforme. Après séchage sur sulfate de magnésium la phase chloroformique est filtrée et concentrée sous vide. L'huile obtenue est reprise dans l'acétate d'isopropyle à chaud. La solution est filtrée à chaud puis refroidie. Le produit obtenu par précipitation est lavé abondamment à l'éther. On obtient finalement 4 g de l-[2-carbéthoxy 4-(5-méthyl 2-thiophéne carboxamido) phénoxy] 3-terbutylamino 2-Propanol sous forme de cristaux blancs fondant à 110°C.

**Exemple 3**

1-[2-carbéthoxy 4-(5-méthyl 2-thiophène carboxamido) phénoxy] 3-isopropylamino 2-propanol.

Formule I $R = C_2H_5$ $R_1 =$ 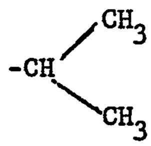 $R_2 = CH_3$ en 5

Le mode opératoire est identique à celui décrit à l'exemple 2, en remplaçant la terbutylamine par l'isopropylamine. On obtient finalement un résidu qui, recristallisé dans l'acétate d'éthyle, fournit 6 g de 1-[2-carbéthoxy 4-(5-méthyl 2-thiophène carboxamido) phénoxy] 3-isopropylamino 2-propanol sous forme de cristaux blancs fondant à 121° C.

**Exemple 4**

1-[2-carbéthoxy 4-(5-chloro 2-thiophène carboxamido) phénoxy] 2,3-époxy propane.
Formule II $R = C_2H_5$ $R_2 = Cl$ en 5
En utilisant le même mode opératoire que celui décrit à l'exemple 1 mais en utilisant 11 g de 2-carbéthoxy 4-(5-chloro 2-thiophène carboxamido) phénol 60 ml d'épichlorhydrine et 1 g de chlorure de benzyltriméthylammonium on obtient 7 g d'une huile qui ne cristallise pas et qui est utilisée ainsi.

**Exemple 5**

Chlorhydrate de 1-[2-carbéthoxy 4-(5-chloro 2-thiophéne carboxamido) phénoxy] 3-terbutylamino 2-propanol.

Formule I $R = C_2H_5$ $R_1 =$ 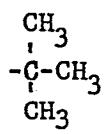 $R_2 = Cl$ en 5

L'huile obtenue à l'exemple 4 est dissoute dans 50 ml de terbutylamine et le mélange porté au reflux pendant 8 h. Le mode opératoire est ensuite le même que celui décrit à l'exemple 2. On obtient les cristaux qui sont repris dans 100 ml d'acétone auxquels sont ajoutés 10 ml d'une solution éthérée d'acide chlorhydrique. Après filtration du précipité et lavage à l'acétone, puis à l'éther, on obtient 700 mg de chlorhydrate de 1-[2-carbéthoxy 4-(5-chloro 2-thiophène carboxamido) phénoxy] 3-terbutylamino 2-propanol sous forme de cristaux blancs fondant à 165° C.

**Exemple 6**

1-[2-carbéthoxy 4-(4-méthyl 2-thiophène carboxamido) phénoxy] 2,3-époxy propane.
Formule II $R = C_2H_5$ $R_2 = CH_3$ en 4
Le mode opératoire est identique à celui décrit à l'exemple 1. En utilisant 30 g de 2-carbéthoxy 4-(4-méthyl 2-thiophène carboxamido) phénol, 3 g de chlorure de benzyl triméthylammonium et 150 ml d'épichlorhydrine, on obtient, après traitement une huile que l'on extrait par deux fois 100 ml d'éther. Après séchage de la phase éthérée et concentration sous vide on obtient 12 g de 1-[2-carbéthoxy 4-(4-méthyl 2-thiophène carboxamido) phénoxy] 2,3-époxy propane sous forme de cristaux blancs fondant à 118° C.

**Exemple 7**

Chlorhydrate de 1-[2-carbéthoxy 4-(4-méthyl 2-thiophène carboxamido) phénoxy] 3-terbutylamino 2-propanol.

Formule I $R = C_2H_5$ $R_1 =$ 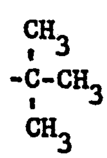 $R_2 = CH_3$ en 4

Le mode opératoire est identique à celui décrit dans l'exemple 2. A partir de 10 g de l'époxyde préparé à l'exemple 6 et 50 ml de terbutylamine on obtient 6 g de cristaux qui sont dissous dans le minimum d'éthanol. Une solution éthérée d'acide chlorhydrique est alors versée jusqu'à obtention d'un pH ~ 1 et le précipité est recueilli par filtration. La recristallisation dans l'éthanol fournit 3 3 g de chlorhydrate de 1-[2-carbéthoxy 4-(4-méthyl 2-thiophène carboxamido) phénoxy] 3-terbutylamino 2-propanol sous forme de cristaux blancs fondant à 186° C.

**Exemple 8**

1-[2-carbéthoxy 4-[2-(4, 5, 6, 7-tétrahydro) thianaphtène carboxamido] phenoxy] 2,3 époxy propane.

Formule II $R = C_2H_5$ $R_2$ = 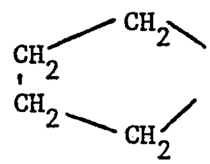 en 4,5

De la même manière à partir du 2-carbéthoxy 4-[2-(4, 5, 6, 7-tétrahydro) thianaphtène carboxamido] phénol de chlorure de benzyltriméthylammonium et d'épichlorhydrine on obtient le 1-[2-carbéthoxy 4-[2-(4, 5, 6, 7-tétrahydro) thianaphtène carboxamido] phénoxy] 2,3-époxy propane.

**Exemple 9**

1-[2-carbéthoxy 4-[2-(4, 5, 6, 7-tétrahydro) thianaphtène carboxamido] phénoxy] 3-terbutylamino 2-propanol.

Formule I $R = C_2H_5$ $R_1 = -\overset{CH_3}{\underset{CH_3}{C}}-CH_3$

$R_2$ = 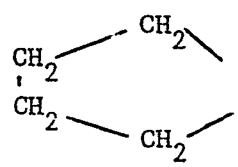 en 3,4

En opérant suivant le mode opératoire décrit à l'exemple 2 mais à partir de l'époxyde obtenu à l'exemple 8 et de terbutylamine on obtient le 1-[2-carbéthoxy 4-[2-(4, 5, 6, 7-tétra-hydro) thianaphtène carboxamido] phénoxy] 3-terbutylamino 2-propanol.

**Exemple 10**

1-[2-carbéthoxy 4-(2-thiényl carboxamido) phénoxy] 2,3-époxy propane
Formule II amide en 2 $R = C_2H_5$
Dans un ballon le mélange de 10 g de 4-(2-thiényl carboxamido) 2-carbéthoxy phénol et 60 ml d'épichlorhydrine est chauffé à 110° C puis 0,9 g de chlorure de benzyltriméthylammonium est ajouté. Le mélange est alors chauffé au reflux pendant 1/2 h puis refroidi. Une fois la température redescendue à 50° C 150 ml d'eau sont introduits. Le mélange réactionnel est bien agité, puis extrait par deux fois 50 ml d'éther. Les phases éthérées sont séchées et évaporées à sec. Le résidu obtenu cristallise dans l'éther et fournit ainsi 8 g de 1-[2-carbéthoxy 4-(2-thiényl carboxamido) phénoxy] 2,3-époxy propane fondant à 110° C.

**Exemple 11**

1-[2-carbéthoxy 4-(3-thiényl carboxamido) phénoxy] 2,3-époxy propane
Formule II amide en 3 $R = C_2H_5$
Selon le mode opératoire décrit dans l'exemple 1 mais à partir de 13 g de 4-(3-thiényl carboxamido) 2-carbéthoxy phénol 70 ml d'épichlorhydrine et 1 g de chlorure de benzyltriméthylammonium on obtient 8 g de 1-[2-carbéthoxy 4-(2-thiényl carboxamido) phénoxy] 2,3-époxy propane fondant à 119° C.

**Exemple 12**

1-[2-carbéthoxy 4-(2-thiényl carboxamido) phénoxy] 3-isopropyl-amino 2-propanol (base)

Formule I amide en 2 $R = C_2H_5$ $R_1 = -\underset{}{\overset{CH_3}{\overset{|}{C}H}}-CH_3$

Dans un ballon et sous agitation sont chauffés, à 50°C 8 g de l'époxyde préparé à l'exemple 1 et 10 ml d'isopropylamine dans 50 ml d'éthanol. Après 8 h de chauffage à 50°C, la solution alcoolique est concentrée sous vide et le résidu repris dans un mélange de 100 ml d'acétate d'isopropyle et 200 ml d'eau. A froid on ajoute alors 3 ml d'acide acétique glacial et on agite jusqu'à l'obtention d'un milieu limpide. L'acétate d'isopropyle est alors éliminé par décantation et la solution acétique basifiée par l'ammoniaque à froid. La phase basique est extraite par 2 fois 50 ml de chloroforme. Après séchage et concentration sous vide de la phase organique on obtient un résidu pâteux qui cristallise dans 50 ml d'éther pour donner 4,5 g de 1-[2-carbéthoxy 4-(2-thiényl carboxamido) phénoxy] 3-isopropylamino 2-propanol fondant à 108°C.

**Exemple 13**

1-[2-carbéthoxy 4-(2-thiényl carboxamido) phénoxy] 3-terbutyl-amino 2-propanol (base)

Formule I amide en 2 $R = C_2H_5$ $R_1 = -\underset{CH_3}{\overset{CH_3}{C}}-CH_3$

Selon le mode opératoire de l'exemple 3 mais à partir de 8 g d'époxyde décrit à l'exemple 1 et 10 ml de terbutylamine on obtient 3,5 g de 1-[2-carbéthoxy 4-(2-thiényl carboxamido) phénoxy] 3-terbutylamino 2-propanol fondant à 125°C.

**Exemple 14**

1-[2-carbéthoxy 4-(3-thiényl carboxamido) phénoxy] 3-isopropyl amino 2-propanol (base)

Formule I amide en 3 $R = C_2H_5$ $R_1 = CH\begin{smallmatrix}CH_3\\CH_3\end{smallmatrix}$

Selon le mode opératoire de l'exemple 3 mais à partir de 8 g de l'époxyde préparé à l'exemple 2 on obtient 5,5 g de 1-[2-carbéthoxy 4-(3-thiényl carboxamido) phénoxy] 3-isopropylamino 2-propanol fondant à 132°C.

**Exemple 15**

1-[2-carbéthoxy 4-(3-thiényl carboxamido) phénoxy] 3-terbutylamino 2-propanol (base)

Formule I amide en 3 $R = C_2H_5$ $R_1 = -\underset{CH_3}{\overset{CH_3}{C}}-CH_3$

Selon le mode opératoire de l'exemple 4 mais à partir de 5,6 g d'époxyde préparé à l'exemple 2 on obtient 3,5 g de 1-[2-carbéthoxy 4-(3-thiényl carboxamido) phénoxy] 3-terbutylamino 2-propanol fondant à 126°C.

**Exemple 16**

1-[2-carbométhoxy 4-(2-thiényl carboxamido) phénoxy] 2,3-époxy propane
Formule II amide en 2 R = $CH_3$

Selon le mode opératoire de l'exemple 1 mais à partir de 35 g de 2-carbométhoxy 4-(2-thiényl carboxamido)phénol, 210 ml d'épichlorhydrine et 3 g de chlorure de benzyltriméthylammonium, on obtient après recristallisation de l'huile brute dans l'acétate d'isopropyle 17 g de 1-[2-carbométhoxy 4-(2-thiényl carboxamido) phénoxy] 2,3-époxy propane fondant à 131°C.

**Exemple 17**

1-[2-carbométhoxy 4-(2-thiényl carboxamido) phénoxy] 3-terbutylamino 2-propanol (base)

Formule I amide en 2 R = $CH_3$ $R_1$ = 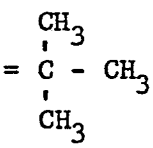

Selon le mode opératoire décrit dans l'exemple 3 mais à partir de 3,6 g de l'époxyde décrit à l'exemple 12 et 10 ml de terbutylamine on obtient 1 g de 1-[2-carbométhoxy 4-(2-thiényl carboxamido) phénoxy] 3-terbutylamino 2-propanol fondant à 136°C.

**TABLEAU**

**PRODUITS DECRITS**

| | |
|---|---|
| Exemple 1 | 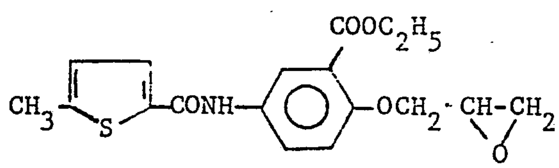 |
| Exemple 2 | 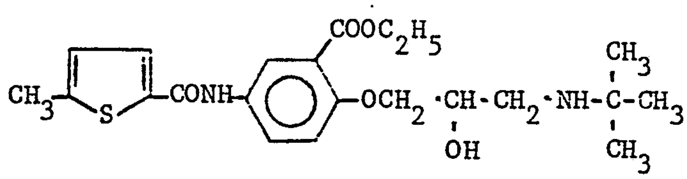 |
| Exemple 3 | 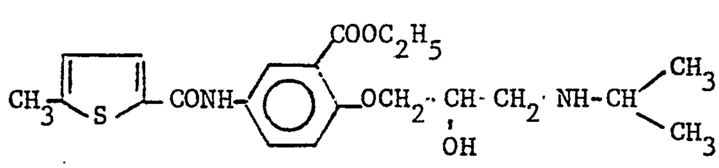 |
| Exemple 4 | 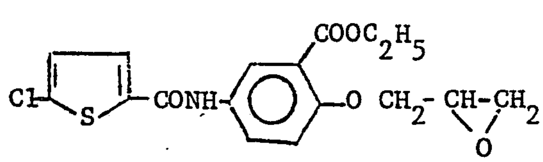 |

**TABLEAU (suite 1)**

| | |
|---|---|
| Exemple 5 | 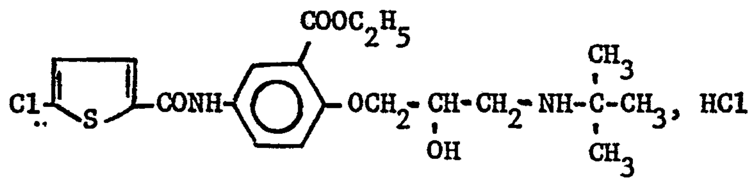 |
| Exemple 6 | 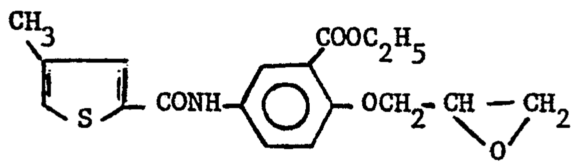 |
| Exemple 7 | 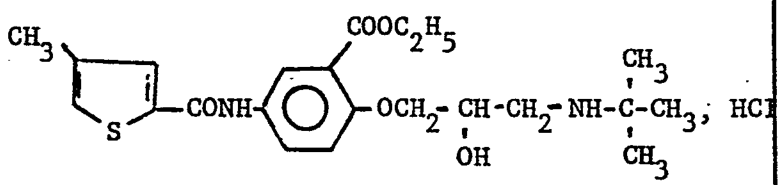 |
| Exemple 8 | 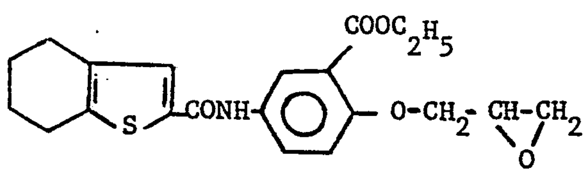 |
| Exemple 9 | 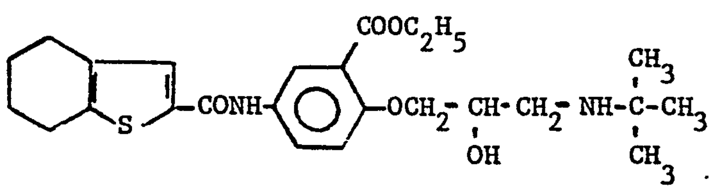 |

**TABLEAU (suite 2)**

| | |
|---|---|
| Exemple 10 | 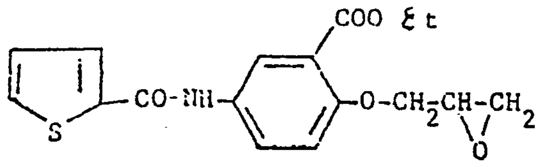 |
| Exemple 11 | 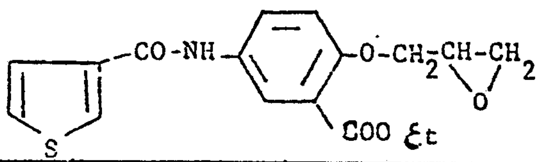 |
| Exemple 12 | 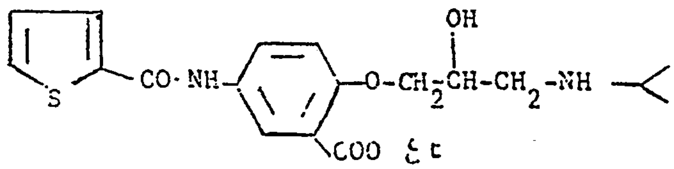 |
| Exemple 13 | 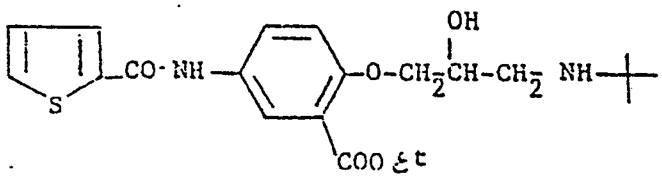 |
| Exemple 14 | 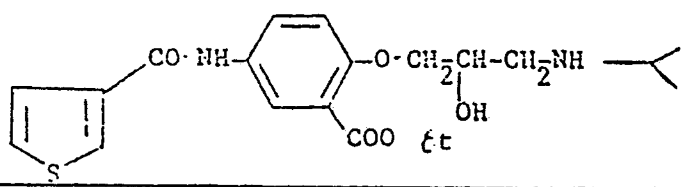 |
| Exemple 15 | 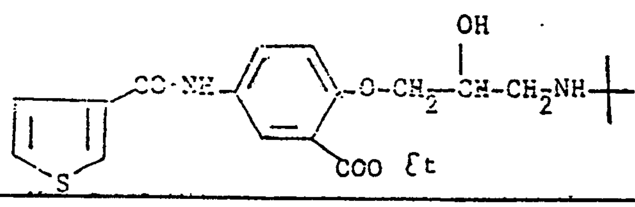 |
| Exemple 16 | 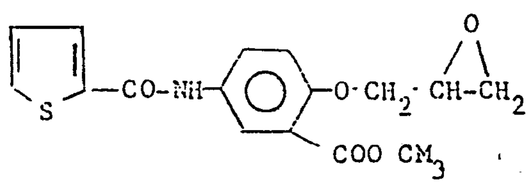 |
| Exemple 17 | 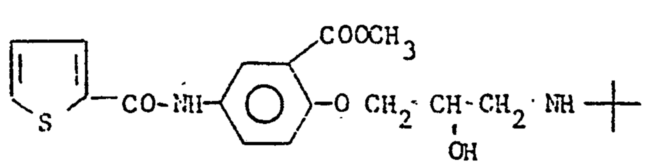 |

**Revendications**

1. Composés caractérisés en ce qu'ils répondent à la formule

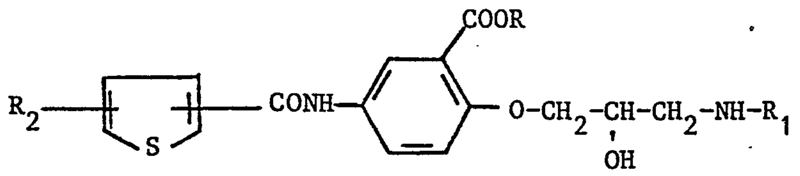 (I)

dans laquelle
- R représente un radical alkyle de 1 à 5 atomes de carbone, ramifié ou non;
- $R_1$ est un radical alkyle de 1 à 5 atomes de carbone, linéaire ou ramifié et,
- $R_2$ peut être H, un radical alkyle de 1 à 5 atomes de carbone, linéaire, ramifié ou cyclique, ou un halogène, $R_2$ peut également désigner un cycle cyclohexyle ou le cyclopentyle accolé au thiophène;
-le groupe carboxamide étant lié au noyau thiophène en position 2 ou 3.

2. Composés selon la revendication 1, caractérisés en ce que R est éthyle.

3. Composés selon l'une des revendications 1 et 2, caractérisés en ce que le radical $R_1$ est isopropyle ou terbutyle.

4. Composés selon la revendication 1, caractérisés en ce que $R_2$ représente le groupe 4-$CH_3$ ou l'atome d'hydrogène.

5. Composés selon la revendication 1, caractérisés en ce que
- la chaîne carboxamide est en position 2 du thiophène; et
- $R_2$ = H ou $CH_3$-4.

6. Composés selon la revendication 1, caractérisés en ce qu'ils sont choisis parmi les suivants:

1-[2-carbéthoxy 4-(5-méthyl 2-thiophène carboxamido) phénoxy] 3-ter-butylamino 2-propanol

Formule I $R = C_2H_5$ $R_1$ = 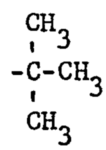 $R_2 = CH_3$ en 5

1-[2-carbéthoxy 4-(5-méthyl 2-thiophène carboxamido) phénoxy] 3-iso-propylamino 2-propanol

Formule I $R = C_2H_5$ $R_1$ = 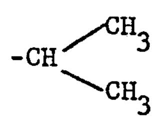 $R_2 = CH_3$ en 5

chlorhydrate de 1-[2-carbéthoxy 4-(5-chloro 2-thiophène carboxamido) phénoxy] 3-terbutylamino 2-propanol

Formule I $R = C_2H_5$ $R_1$ = 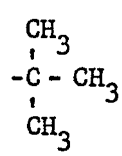 $R_2 = Cl$ en 5

chlorhydrate de 1-[2-carbéthoxy 4-(4-méthyl 2-thiophène carboxamido) phénoxy] 3-terbutylamino 2-propanol

Formule I R = $C_2H_5$ $R_1$ = 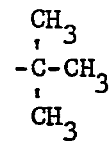 $R_2 = CH_3$ en 4

1-[2-carbéthoxy 4-[2-(4, 5, 6, 7-tétrahydro) thianaphtène carboxamido] phénoxy] 3-terbutylamino 2-propanol

Formule I R = $C_2H_5$ $R_1$ = 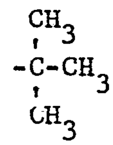 $R_2$ = 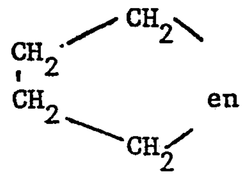 en 3, 4

1-[2-carbéthoxy 4-(2-thiényl carboxamido) phénoxy] 3-isopropyl amino 2-propanol (base)

Formule I amide en 2 R = $C_2H_5$ $R_1$ = 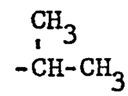

1-[2-carbéthoxy 4-(2-thiényl carboxamido) phénoxy] 3-terbutyl amino 2-propanol (base)

Formule I amide en 2 R = $C_2H_5$ $R_1$ = 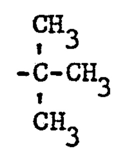

1-[2-carbéthoxy 4-(3-thiényl carboxamido) phénoxy] 3-isopropyl amino 2-propanol (base)

Formule I amide en 3 R = $C_2H_5$ $R_1$ = 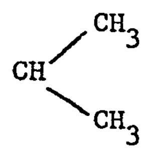

1-[2-carbéthoxy 4-(3-thiényl carboxamido) phénoxy] 3-terbutylamino 2-propanol (base)

Formule I amide en 3 R = $C_2H_5$ $R_1$ = 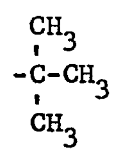

1-[2-carbométhoxy 4-(2-thiényl carboxamido) phénoxy] 3-terbutyl-amino 2-propanol (base)

Formule I amide en 2 R = $CH_3$ $R_1$ = 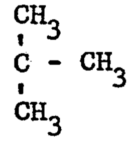

7. Procédé de préparation des composés selon l'une quelconque des revendicationsl à 6 caractérisé en ce qu'on fait réagir une base:

$NH_2-R_1$

dans laquelle $R_1$ est tel que défini dans l'une quelconque des revendications 1 à 6, sur un composé de formule

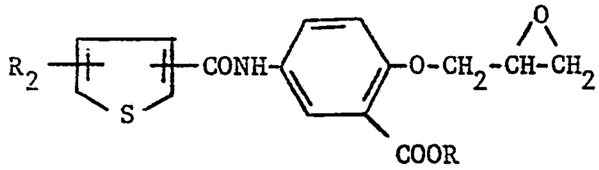

(II)

dans laquelle R et $R_2$ sont tels que définis dans l'une quelconque des revendications 1 à 6 sans solvant ou dans un solvant organique usuel comme les alcools, à 20-150°C.

8. Procédé selon la revendication 7, caractérisé en ce que les composés de formule II sont préparés par réaction d'un phénol de formule:

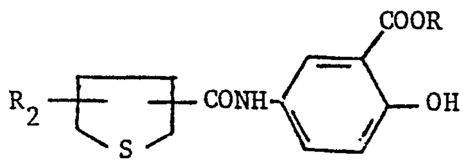

(III)

dans laquelle R et $R_2$ sont tels que définis dans l'une quelconque des revendications 1 à 6, sur un excès d'épichlorhydrine, en présence d'un catalyseur comme le benzyltriméthylammonium, à 110-130°C.

9. Intermédiaires pour la synthèse des composés selon l'une quelconque des revendications 1 à 6, caractérisés en ce qu'ils répondent à la formule

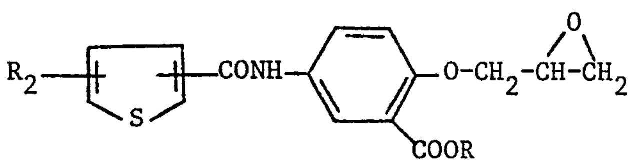

(II)

dans laquelle

- R représente un radical alkyle de 1 à 5 atomes de carbone, ramifié ou non;
- $R_2$ représente un radical alkyle de 1 à 5 atomes de carbone, linéaire, ramifié ou cyclique, ou un halogène et $R_2$ peut également désigner un cycle accolé au thiophène cyclohexyle ou cyclopentyle;

le groupe carboxamide étant lié au noyau thiophène en position 2 ou 3.

10. Intermédiaires pour la synthèse des composés selon l'une quelconque des revendications 1 à 6, caractérisés en ce qu'ils répondent à la formule

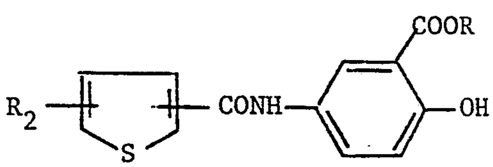

(III)

dans laquelle:

- R représente un radical alkyle de 1 à 5 atomes de carbone, ramifié ou non;
- $R_2$ représente un radical alkyle de 1 à 5 atomes de carbone, linéaire, ramifié ou cyclique, ou un halogène et $R_2$ peut également désigner un cycle accolé au thiophène cyclohexyle ou cyclopentyle;
- le groupe carboxamide étant lié au groupe thiophène en position 2 ou 3.

11. Médicaments utiles comme β-bloquants et diurétiques, caractérisés en ce qu'ils contiennent au moins un composé selon la revéndication 1.

12. Médicaments utiles simultanément comme diurétiques et β-bloquants, caractérisés en ce qu'ils contiennent au moins un composé selon la revendication 6.

**Patentansprüche**

1. Verbindungen, dadurch gekennzeichnet, daß sie der Formel

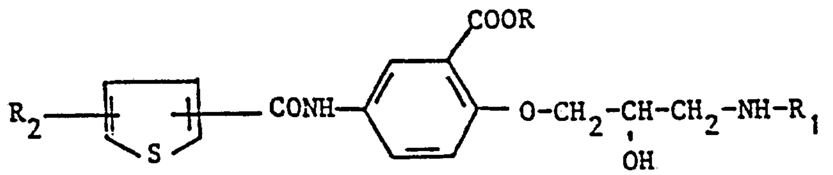

(I)

entsprechen, worin

- R eine verzweigte oder nichtverzweigte Alkylgruppe mit 1 bis 5 Kohlenstoffatomen darstellt;
- $R_1$ eine gerade oder verzweigte Alkylgruppe mit 1 bis 5 Kohlenstoffatomen bedeutet;
- $R_2$ H, eine gerade, verzweigte oder zyklische Alkylgruppe mit 1 bis 5 Kohlenstoffatomen oder ein Halogen sein kann, wobei $R_2$ auch einen an Thiophen kondensierten Cyclohexyl- oder Cyclopentylring bezeichnen kann;
- wobei die Carboxamidgruppe an den Thiophenkern in der Position 2 oder 3 gebunden ist.

2. Verbindungen nach Anspruch 1, dadurch gekennzeichnet, daß R Äthyl ist.

3. Verbindungen nach einem der Ansprüche 1 und 2, dadurch gekennzeichnet, daß die Gruppe $R_1$ Isopropyl oder tert. Butyl ist.

4. Verbindungen nach Anspruch 1, dadurch gekennzeichnet, daß $R_2$ die Gruppe 4-$CH_3$ oder ein Wasserstoffatom bezeichnet.

5. Verbindungen nach Anspruch 1, dadurch gekennzeichnet, daß

- sich die Carboxamidkette an der Position 2 des Thiophens befindet; und
- $R_2$ = H oder 4-$CH_3$.

6. Verbindungen nach Anspruch 1, dadurch gekennzeichnet, daß sie ausgewählt sind aus den folgenden:

1-[2-Carbäthoxy-4-(5-methyl-2-thiophencarboxamido)-phen-oxy]-3-tert.butylamino-propan-2-ol

Formel I   R = $C_2H_5$   $R_1$ = 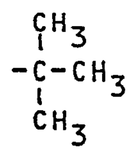   $R_2 = CH_3$ in 5

1-[2-Carbäthoxy-4-(5-methyl-2-thiophencarboxamido)-phenoxy] -3-isopropylamino-propan-2-ol

Formel I   R = $C_2H_5$   $R_1$ = 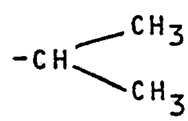   $R_2 = CH_3$ in 5

1-[2-Carbäthoxy-4-(5-chlor-2-thiophencarboxamido)-phen-oxy]-3-tert.butylamino-propan-2-ol-hydrochlorid

Formel I   R = $C_2H_5$   $R_1$ = 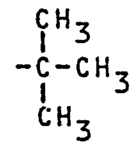   $R_2$ = Cl in 5

1-[2-Carbäthoxy-4-(4-methyl-2-thiophencarboxamido)-phen-oxy]-3-tert.butylamino-propan-2-ol-hydrochlorid

Formel I $R = C_2H_5$ $R_1 =$ 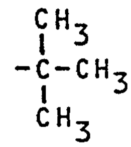 $R_2 = CH_3$ in 4

1-[2-Carbäthoxy-4-[2-(4, 5, 6, 7-tetrahydro)-thianaphthen-carboxamido]-phenoxy]-3-tert.butylamino-propan-2-ol

Formel I $R = C_2H_5$ $R_1 =$ 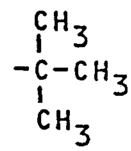 $R_2 =$ 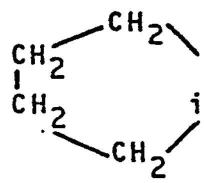 in 3,4

1-[2-Carbäthoxy-4-(2-thienylcarboxamido)-phenoxy]-3-iso-propylamino-propan-2-ol (Base)

Formel I Amid in 2 $R = C_2H_5$ $R_1 =$ 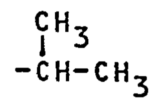

1-[2-Carbäthoxy-4-(2-thienylcarboxamido)-phenoxy] -3-tert.butylamino-propan-2-ol (Base)

Formel I Amid in 2 $R = C_2H_5$ $R_1 =$ 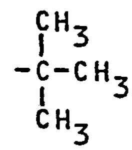

1-[2-Carbäthoxy-4-(3-thienylcarboxamido)-phenoxy] -3-iso-propylamino-propan-2-ol (Base)

Formel I Amid in 3 $R = C_2H_5$ $R_1 =$ 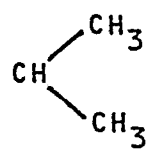

1-[2-Carbäthoxy-4-(3-thienylcarboxamido)-phenoxy] -3-tert.butylamino-propan-2-ol (Base)

Formel I Amid in 3 $R = C_2H_5$ $R_1 =$ 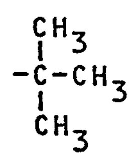

1-[2-Carbomethoxy-4-(2-thienylcarboxamido)-phenoxy]-3-tert.butylamino-propan-2-ol (Base)

Formel I Amid in 2 $R = CH_3$ $R_1 =$ 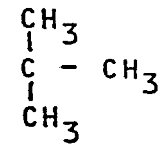

7. Verfahren zur Herstellung der Verbindungen nach einem der Ansprüche 1 bis 6, dadurch gekennzeichnet, daß man eine Base

$NH_2$-$R_1$,

worin $R_1$ wie in einem der Ansprüche 1 bis 6 definiert ist, mit einer Verbindung der Formel

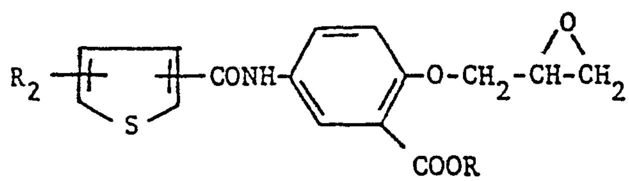 (II)

worin R und $R_2$ wie in einem der Ansprüche 1 bis 6 definiert sind, ohne Lösungsmittel oder in einem üblichen organischen Lösungsmittel, wie einem Alkohol, bei 20 bis 150° C zur Umsetzung bringt.

8. Verfahren nach Anspruch 7, dadurch gekennzeichnet, daß die Verbindungen der Formel II hergestellt werden durch Umsetzung eines Phenols der Formel

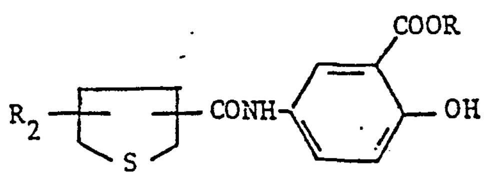 (III)

worin R und $R_2$ wie in einem der Ansprüche 1 bis 6 definiert sind, mit einem Überschuß an Epichlorhydrin in Gegenwart eines Katalysators, wie Benzyltrimethylammonium bei 110 bis 130° C.

9. Zwischenprodukte für die Synthese der Verbindungen nach einem der Anspruche 1 bis 6, dadurch gekennzeichnet, daß sie der Formel

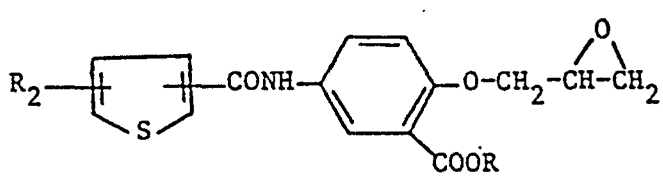 (II)

entsprechen, worin

- R eine verzweigte oder nichtverzweigte Alkylgruppe mit 1 bis 5 Kohlenstoffatomen darstellt;
- $R_2$ eine gerade, verzweigte oder zyklische Alkylgruppe mit 1 bis 5 Kohlenstoffatomen oder ein Halogen darstellt und $R_2$ auch ein an Thiophen kondensierter Cyclohexyl- oder Cyclopentylring sein kann;
- wobei die Carboxamidgruppe an den Thiophenkern in der Position 2 oder 3 gebunden ist.

10. Zwischenprodukte für die Synthese der Verbindungen nach einem der Ansprüche 1 bis 6, dadurch gekennzeichnet, daß sie der Formel

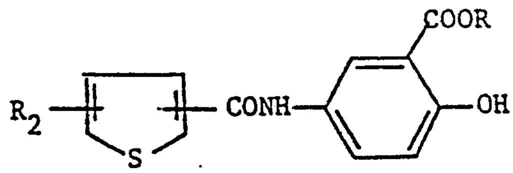

(III)

entsprechen, worin

- R eine verzweigte oder nichtverzweigte Alkylgruppe mit 1 bis 5 Kohlenstoffatomen darstellt;
- $R_2$ eine gerade, verzweigte oder zyklische Alkylgruppe mit 1 bis 5 Kohlenstoffatomen oder ein Halogen darstellt und $R_2$ auch einen an Thiophen kondensierten Cyclohexyl- oder Cyclopentylring bezeichnen kann;
- wobei die Carboxamidgruppe an den Thiophenkern in der Position 2 oder 3 gebunden ist.

11. Medikamente, die als β-Blocker und Diuretika nützlich sind, dadurch gekennzeichnet, daß sie mindestens eine Verbindung nach Anspruch 1 enthalten.

12. Medikamente, die gleichzeitig als Diuretika und β-Blocker nützlich sind, dadurch gekennzeichnet, daß sie mindestens eine Verbindung nach Anspruch 6 enthalten.

**Claims**

1. Compounds characterized in that they correspond to formula

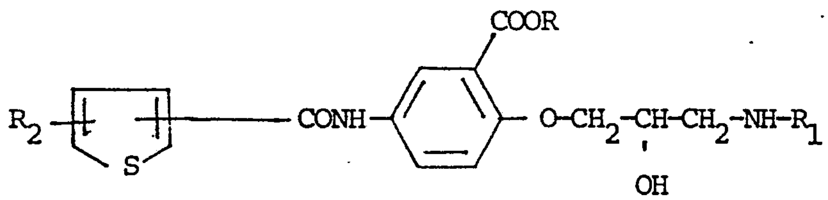

(I)

in which:

- R represents an alkyl radical of 1 to 5 carbon atoms, branched or not;
- $R_1$ is an alkyl radical of 1 to 5 carbon atoms, straight or branched;
- $R_2$ may be H, an alkyl radical of 1 to 5 carbon atoms, straight, branched or cyclic, or a halogen, $R_2$ may also designate a cycle joined to the thiophene such as cyclohexyl or cyclopentyl; the carboxamide group being linked to the thiophene nucleus in 2 or 3 position.

2. Compounds according to claim 1, characterized in that R is ethyl.

3. Compounds according to any one of claims 1 and 2, characterized in that radical $R_1$ is isopropyl or terbutyl.

4. Compounds according to claim 1, characterized in that $R_2$ represents the 4-$CH_3$ group or the hydrogen atom.

5. Compounds according to claim 1, characterized in that

- the carboxamide chain is in 2 position of the thiophene; and
- $R_2$ = H or $CH_3$-4.

6. Compounds according to claim 1, characterized in that they are selected from the following: 1-[2-carbethoxy 4-(5-methyl2-thiophene carboxamido)phenoxy] 3-terbutylamino 2-propanol

$CH_3$
Formula I R = $C_2H_5$ $R_1$ = $-C-CH_3$ $R_2$ = $CH_3$ in 5
$CH_3$

1-[2-carbethoxy 4-(5-methyl 2-thiophene carboxamido) phenoxy] 3-isopropylamino 2-propanol

Formula I R = $C_2H_5$ $R_1$ = 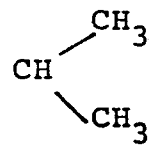 $R_2 = CH_3$ in 5

hydrochloride of 1-[2-carbethoxy 4-(5-chloro 2-thiophene carboxamido) phenoxy] 3-terbutylamino 2-propanol.

Formula I R = $C_2H_5$ $R_1$ = 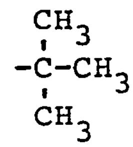 $R_2$ = Cl in 5

hydrochloride of 1-[2-carbethoxy 4-(4-methyl 2-thiophene carboxamido)phenoxy] 3-terbutylamino 2-propanol

Formula I R = $C_2H_5$ $R_1$ = 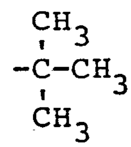 $R_2 = CH_3$ in 4

1-[carbethoxy 4-[2-(4, 5, 6, 7,-tetrahydro) thianaphthene carboxamido] phenoxy] 3-terbutylamino 2-propanol

Formula I R = $C_2H_5$ $R_1$ = 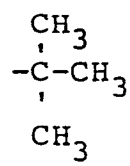 $R_2$ = 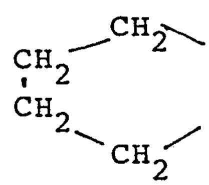 in 3,4

1-[2-carbethoxy 4-(2-thienyl carboxamido) phenoxy] 3-iso-propyl amino 2-propanol (base)

Formula I amide in 2 R= $C_2H_5$ $R_1$ = 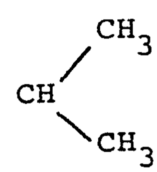

1-[2-carbethoxy 4-(2-thienyl carboxamido) phenoxy] 3-ter-butyl amino 2-propanol (base)

Formula I amide in 2 R = $C_2H_5$ $R_1$ = 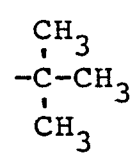

1-[2-carbethoxy 4-(3-thienyl carboxamido) phenoxy] 3-iso-propyl amino 2-propanol (base)

Formula I amide in 3 R = $C_2H_5$ $R_1$ = 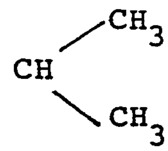

1-[2-carbethoxy 4-(3-thienyl carboxamido) phenoxy] 3-ter-butylamino 2-propanol (base)

Formula I amide in 3 R = $C_2H_5$ $R_1$ = 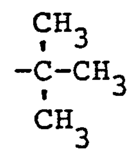

1-[2-carbomethoxy 4-(2-thienyl carhoxamido) phenoxy] 3-terbutylamino 2-propanol (base)

Formula I amide in 2 R = $CH_3$ $R_1$ = 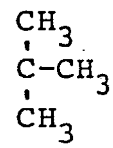

7. Process for preparing the compounds according to any one of claims 1 to 6, characterized in that it comprises the step of reactińg a base;

$NH_2$-$R_1$

in which $R_1$ is as defined in any one of claims 1 to 6, on a compound of formula

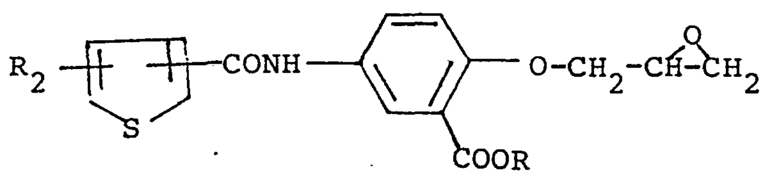

(II)

in which R and $R_2$ are as defined in any one of claims 1 to 6, without solvent or in a conventional organic solvent such as alcohols, at 20 - 150° C.

8. The process according to claim 7, characterized in that the compounds of formula II are prepared by reaction of a phenol of formula:

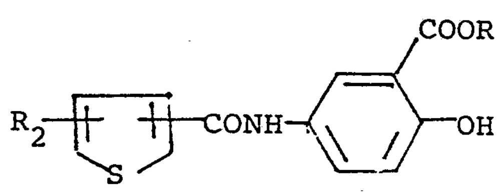

(III)

in which R and $R_2$ are as defined in any one of claims 1 to 6, on an excess of epichlorohydrin, in the presence of a catalyst such as benzyltrimethylammonium, at 110-130° C.

9. Intermediaries for the synthesis of the compounds according to any one of claims 1 to 6, characterized in that they correspond to formula:

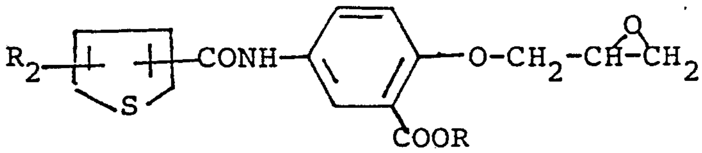

(II)

in which:

- R represents an alkyl radical of 1 to 5 carbon atoms, branched or not;
- $R_2$ represents an alkyl radical of 1 to 5 carbon atoms, straight, branched or cyclic, or a halogen and $R_2$ may also designate a cyclohexyl or cyclopentyl cycle joined to the thiophene; the carboxamide group being linked to the thiophene nucleus in 2 or 3 position.

10. Intermediaries for the synthesis of the compounds according to any one of claims 1 to 6, characterized in that they correspond to formula:

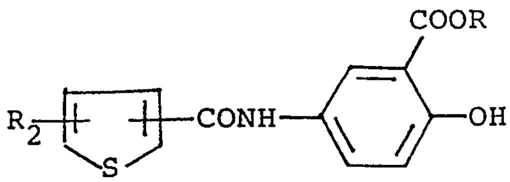

(III)

in which:

- R represents an alkyl radical of 1 to 5 carbon atoms, branched or not;
- $R_2$ represents an alkyl radical of 1 to 5 carbon atoms, straight, branched or cyclic, or a halogen and $R_2$ may also designate a cyclohexyl or cyclopentyl cycle joined to the thiophene; the carboxamide group being linked to the thiophene nucleus in 2 or 3 position.

11. Drugs useful as β-blocking and/or diuretic substances, characterized in that they contain at least one compound according to claim 1.

12. Drugs useful simultaneously as diuretics and β-blocking substances, characterized in that they contain at least one compound according to claim 6.